(19) Europäisches Patentamt
European Patent Office
Office européen des brevets

(11) Veröffentlichungsnummer: **0 293 743 B1**

# (12) EUROPÄISCHE PATENTSCHRIFT

(45) Veröffentlichungstag der Patentschrift: **14.08.91**

(51) Int. Cl.5: **C07D 403/04**, A01N 43/56, A01N 43/54

(21) Anmeldenummer: **88108360.4**

(22) Anmeldetag: **26.05.88**

## (54) Substituierte 5-Amino-Pyrazole und diese enthaltende Fungizide.

(30) Priorität: **03.06.87 DE 3718526**

(43) Veröffentlichungstag der Anmeldung:
**07.12.88 Patentblatt 88/49**

(45) Bekanntmachung des Hinweises auf die Patenterteilung:
**14.08.91 Patentblatt 91/33**

(84) Benannte Vertragsstaaten:
**AT BE CH DE ES FR GB GR IT LI NL SE**

(56) Entgegenhaltungen:
**EP-A- 0 183 159**
**EP-A- 0 212 281**
**DE-A- 3 419 127**
**DE-B- 1 220 428**
**US-A- 3 040 047**

(73) Patentinhaber: **BASF Aktiengesellschaft**
**Carl-Bosch-Strasse 38**
**W-6700 Ludwigshafen(DE)**

(72) Erfinder: **Graf, Hermann, Dr.**
**Ginsterstrasse 15**
**W-6704 Mutterstadt(DE)**
Erfinder: **Schuetz, Franz, Dr.**
**Budapester Strasse 45**
**W-6700 Ludwigshafen(DE)**
Erfinder: **Sauter, Hubert, Dr.**
**Neckarpromenade 20**
**W-6800 Mannheim 1(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

EP 0 293 743 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, substituierte 5-Amino-pyrazole der Formel I mit fungizider Wirkung, Verfahren zu ihrer Herstellung und diese als Wirkstoffe enthaltende Fungizide.

Die substituierten 5-Amino-pyrazole können durch die allgemeine Formel

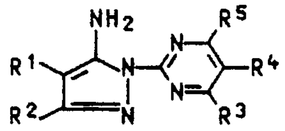

(I),

in der

$R^1$ einen geradkettigen oder verzweigten $C_4$-$C_{12}$-Alkyl- oder einen geradkettigen oder verzweigten $C_4$-$C_{12}$-Alkoxy-$C_1$-$C_5$-alkylrest oder einen gegebenenfalls durch $C_1$-$C_4$-Alkyl oder Halogen im Phenylrest oder Naphthylrest substituierten Phenoxy-$C_1$-$C_8$-alkylrest oder Naphthoxy-$C_1$-$C_8$-alkylrest,

$R^2$ $R^3$ und $R^5$ unabhängig voneinander Wasserstoff oder einen $C_1$-$C_4$-Alkyl-rest,

$R^4$ Wasserstoff, einen geradkettigen oder verzweigten $C_1$-$C_6$-Alkyl- oder einen geradkettigen oder verzweigten $C_1$-$C_6$-Alkoxy-$C_1$-$C_5$-alkylrest bedeuten,

wiedergegeben werden.

In Formel I bedeutet $R^1$ $C_4$-$C_{12}$-Alkyl, vorzugsweise $C_6$-$C_{10}$-Alkyl, beispielsweise geradkettiges oder verzweigtes Butyl, Pentyl, Hexyl, Heptyl, Octyl, Nonyl, Decyl, Undecyl oder Dodecyl, oder $C_4$-$C_{12}$-Alkoxy-$C_1$-$C_5$-alkyl, vorzugsweise $C_6$-$C_{10}$-Alkoxy-$C_1$-$C_5$-alkyl, beispielsweise durch geradkettiges oder verzweigtes Butoxy, Pentyloxy, Hexyloxy, Heptyloxy, Octyloxy, Nonyloxy, Decyloxy, Undecyloxy oder Dodecyloxy substituiertes $C_1$-$C_5$-Alkyl, wie durch diese Alkoxyreste substituiertes Methyl, Ethyl, n-Propyl, i-Propyl, n-Butyl. Die $C_1$-$C_5$-Alkylreste sind vorzugsweise in $\omega$-Stellung durch Alkoxy substituiert.

$R^1$ kann außerdem einen Phenoxy-$C_1$-$C_8$-alkylrest oder Naphthoxy-$C_1$-$C_8$-alkyrest, vorzugsweise einen Phenoxy-$C_2$-$C_6$-alkylrest oder Naphthoxy-$C_2$-$C_6$-alkylrest bedeuten. Naphthoxy oder Phenoxy, vorzugsweise Phenoxy, kann einen oder mehrere Halogen- oder $C_1$-$C_4$-Alkyl-Substituenten, wie Chlor oder Methyl, tragen. Beispiele für Rest $R^1$ sind durch Phenoxy, 1-Naphthoxy, 2-Naphthoxy, o-, m- oder p-Kresyl, 2,3-, 2,4-, 2,5-, 2,6-, 3,4- oder 3,5-Xylenyl, 2-, 3- oder 4-Chlorphenoxy oder 2,4- oder 3,5-Dichlorphenoxy substituierte Alkylreste, wie Methyl, Ethyl, n-Propyl, i-Propyl, n-Butyl, sec-Butyl oder n-Pentyl. Die Alkylreste sind vorzugsweise in $\omega$-Stellung durch Aryloxy substituiert.

Beispiele für geradkettige und verzweigte Alkylsubstituenten $R^2$ $R^3$ $R^4$ und $R^5$ in Formel I sind je nach C-Zahl Methyl, Ethyl, n-Propyl, i-Propyl, n-Butyl, i-Butyl, geradkettiges oder verzweigtes Pentyl oder Hexyl. Bevorzugter Alkylrest ist Methyl.

$C_1$-$C_6$-Alkoxy-$C_1$-$C_5$-alkylreste für $R^4$ können durch Methoxy, Ethoxy, n-Propoxy, i-Propoxy, geradkettiges oder verzweigtes Butoxy, Pentoxy oder Hexoxy substituierte Methyl-, Ethyl-, n-Propyl-, i-Propyl-, n-Butyl-, n-Pentyl-, i-Pentyl-, sec-Pentyl-, tert.-Pentyl- oder neo-Pentylreste sein. Bevorzugt sind auch hier in $\omega$-Stellung durch Alkoxy substituierte Alkylreste.

Bevorzugte Verbindungen der Formel I sind solche, bei denen $R^4$ $R^4$ und $R^5$ Wasserstoff bedeuten.

Die substituierten 5-Amino-pyrazole der Formel I lassen sich durch Umsetzung von $\alpha$-Acyl-nitrilen der Formel IIa bzw. IIb, wobei IIb als syn-oder als anti-Isomeres oder als Gemisch von beiden vorliegen kann, gegebenenfalls im Gemisch mit IIa,

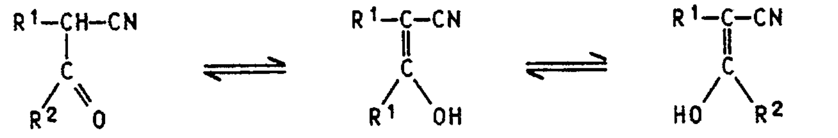

IIa IIb (syn) IIb (anti)

mit 2-Hydrazino-pyrimidinen der Formel III

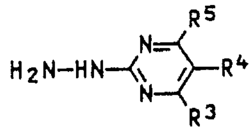

(III)

in einem geeigneten Lösungs- oder Verdünnungsmittel und gegebenenfalls unter Zusatz eines Katalysators herstellen. In den Formeln II und III haben die Substituenten $R^1$ $R^2$ $R^3$ $R^4$ und $R^5$ die für Formel I angegebenen Bedeutungen.

Die Herstellung von 5-Amino-pyrazolen aus α-Acyl-nitrilen und Hydrazinen ist an sich bekannt (J. Prakt. Chem. [2] 90, 223 (1914); J. Chem. Soc. 1941, 285).

Als Lösungs- oder Verdünnungsmittel für die Kondensation von II mit III kommen Benzol, Chlorbenzol, Toluol, Xylol, Ethylbenzol oder Dibutylether in Betracht. Vorteilhafterweise wird das während der Reaktion entstehende Wasser ausgekreist.

Außerdem kann es von Vorteil sein, der Reaktionsmischung einen Katalysator, wie Benzolsulfonsäure, p-Toluolsulfonsäure oder p-Phenolsulfonsäure, in einer Menge von 0,01 % bis 10 %, bezogen auf Verbindung III, zuzusetzen.

Die Reaktionstemperatur wird durch die Siedetemperatur des entsprechenden Lösungsmittel-Wasser-Azeotrops bestimmt.

Nach Ende der Reaktion wäscht man die Reaktionslösung mit Wasser oder wäßriger Carbonatlösung säurefrei, trocknet und dampft ein. Eine Reinigung des Eindampfrückstandes durch Kristallisation kann in den meisten Fällen entfallen.

Die α-Acyl-nitrile II können, soweit sie nicht bekannt sind, nach bekannten Methoden aus Nitrilen mit α-ständigem Wasserstoff und Carbonsäureestern mit starken Basen, z.B. Alkalihydriden, Alkaliamiden oder Metallalkylen, hergestellt werden (J. Amer. Chem. Soc. 73 (1951), S. 3766; Houben-Weyl, Methoden der Organ. Chemie, Bd. 8, S. 573 ff. (1952); Bd. E3, S. 6 ff. (1983); DE-A-27 53 322).

Die Herstellung der 2-Hydrazino-pyrimidine III kann nach Bull. Soc. Chim. Belg. 68, 30 - 46 (1959) durch Umsetzung der entsprechenden 2-Chlor- oder 2-Mercapto-pyrimidine mit Hydrazin-hydrat erfolgen.

Herstellungsbeispiele

I. 5-Amino-4-n-nonyl-1-(2-pyrimidinyl)-1H-pyrazol (Verbindung Nr. 42)

a) 2-Hydrazino-pyrimidin-hydrat

114 g (1,00 mol) 2-Chlor-pyrimidin werden in 500 ml Methanol mit 200 ml 100 proz. (4,12 mol) Hydrazinhydrat versetzt. Nach Abklingen der leichten Erwärmung rührt man über Nacht nach, filtriert den entstandenen Niederschlag ab, wäscht mit Pentan nach und trocknet. Einengen der Mutterlauge ergibt weiteres Material, das wie oben behandelt wird. Gesamtausbeute 86,9 g (68 %), Schmp. 81-83 °C.

b) 2-Formyl-laurinsäure-nitril

50,1 g (300 mmol) Laurinsäurenitril werden in 300 ml trockenem Tetrahydrofuran gelöst, auf -60 °C abgekühlt, binnen 1 h mit 220 ml einer 1,5 M Lösung Butyllithium in n-Hexan (entspricht 330 mmol) versetzt und 4 h bei -60 °C nachgerührt. Anschließend tropft man 22,2 g (300 mmol) Ethylformiat, gelöst in 150 ml Tetrahydrofuran, zu und hält danach 3 h bei -60 °C. Über Nacht läßt man langsam auf Raumtemperatur kommen. Man versetzt vorsichtig unter Kühlung mit wäßriger Salzsäure, bis ein pH von vier erreicht ist, trennt die Phasen, wäscht die organ. mehrmals mit Wasser, trocknet und engt ein. 58 g eines gelben Öls bleiben zurück, das nach GC-Analyse zu 81 % aus dem gewünschten Produkt besteht.

c) 5-Amino-4-n-nonyl-1-(2-pyrimidinyl)-1H-pyrazol

24,0 g 81 proz. Produkt aus b) (entspricht 19,5 g Reinprodukt, 100 mmol), 12,1 g (101 mmol) Hydrazin aus a) und eine Spatelspitze p-Toluolsulfonsäure werden in 200 ml Toluol unter Erhitzen im Wasserauskreiser zur Reaktion gebracht. Geht kein Wasser mehr über (nach ca. 4 h), läßt man abkühlen und filtriert den entstandenen Niederschlag ab, wäscht mit Wasser und Petrolether nach und trocknet. Einengen der Mutterlauge ergibt weiteres Material. Ausbeute 15,5 g (54 %), Schmp. 122-123 °C. Die Substanz ist laut dünnschichtchromatographischer Analyse einheitlich.

II. 5-Amino-4-n-hexyl-1-(4,6-dimethyl-pyrimidin-2-yl)-1H-pyrazol (Verbindung Nr. 128)

a) 2-Hydrazino-4,6-dimethyl-pyrimidin

122,7 g (876 mmol) 2-Mercapto-4,6-dimethylpyrimidin werden mit 175 g (3,5 mol) Hydrazinhydrat in 1,0 l Methanol 12 h bei Raumtemp., dann 8 h unter Rückfluß zur Reaktion gebracht. Nach Abkühlen

wird der entstandene Niederschlag abgesaugt, gewaschen und getrocknet. Ausbeute 63,0 g (52 %), Schmp. 161-162 °C.

b) 2-Formyl-caprylsäure-nitril

100 g (800 mmol) Caprylsäurenitril werden in 300 ml trockenem Tetrahydrofuran gelöst, auf -60 °C abgekühlt, langsam mit 586 ml 1,5 M n-Butyllithium in n-Hexan (880 mmol) versetzt und 4 h bei -60 °C nachgerührt. Anschließend gibt man 59,2 g (800 mmol) Methylformiat, gelöst in 150 ml Tetrahydrofuran, zu und läßt 3 h bei -60 °C reagieren. Danach wird die Kühlung entfernt. Wenn wieder Raumtemp. erreicht ist, wird unter Kühlung mit verd. Salzsäure auf pH 4 eingestellt, die Phasen getrennt, die organische mit Wasser gewaschen, getrocknet und eingeengt. Der Rückstand (126 g) besitzt laut GC-Analyse einen Gehalt an Produkt von 73 %.

c) 5-Amino-4-n-hexyl-1-(4,6-dimethyl-pyrimidin-2-yl)-1H-pyrazol

20,9 g 73 proz. Produkt aus b) (entspricht 15,3 g Reinprodukt, 100 mmol), 13,8 g (100 mmol) Hydrazin aus a) und eine Spatelspitze p-Toluolsulfonsäure erhitzt man in 250 ml Toluol unter Auskreisung von Wasser. Nach 6 h ist die Wasserabscheidung beendet. Man läßt abkühlen, filtriert, wäscht mit Wasser, trocknet und engt ein. Das zurückbleibende Öl reibt man mit Pentan an. Ausbeute nach Trocknen 12,2 g (45 %), Schmp. 101-102 °C, dünnschichtchromatographisch einheitlich.

Analog dem in den Herstellungsbeispielen beschriebenen Verfahren können die in den nachstehenden Tabellen näher charakterisierten Wirkstoffe hergestellt werden. Die nicht näher charakterisierten Verbindungen können unter entsprechender Abwandlung der Rohstoffe und Anpassung der Herstellvorschriften leicht erhalten werden.

Tabelle 1

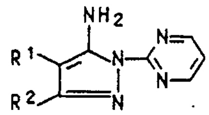

| Verb.Nr. | $R^1$ | $R^2$ | Schmp. (°C) |
|---|---|---|---|
| 1 | $n-C_4H_9$ | H | |
| 2 | $i-C_4H_9$ | H | |
| 3 | $s-C_4H_9$ | H | |
| 4 | $t-C_4H_9$ | H | |
| 5 | $n-C_4H_9$ | $CH_3$ | |
| 6 | $i-C_4H_9$ | $CH_3$ | |
| 7 | $s-C_4H_9$ | $CH_3$ | |
| 8 | $t-C_4H_9$ | $CH_3$ | |
| 9 | $n-C_4H_9$ | $C_2H_5$ | |
| 10 | $i-C_4H_9$ | $C_2H_5$ | |
| 11 | $s-C_4H_9$ | $C_2H_5$ | |
| 12 | $t-C_4H_9$ | $C_2H_5$ | |
| 13 | $n-C_4H_9$ | $n-C_3H_7$ | |
| 14 | $i-C_4H_9$ | $n-C_3H_7$ | |
| 15 | $s-C_4H_9$ | $n-C_3H_7$ | |
| 16 | $t-C_4H_9$ | $n-C_3H_7$ | |
| 17 | $n-C_4H_9$ | $n-C_4H_9$ | |
| 18 | $i-C_4H_9$ | $n-C_4H_9$ | |
| 19 | $n-C_4H_9$ | $i-C_4H_9$ | |
| 20 | $i-C_4H_9$ | $i-C_4H_9$ | |
| 21 | $n-C_5H_{11}$ | H | |
| 22 | $n-C_5H_{11}$ | $CH_3$ | |
| 23 | $n-C_5H_{11}$ | $C_2H_5$ | |
| 24 | $i-C_5H_{11}$ | H | |
| 25 | $i-C_5H_{11}$ | $CH_3$ | |
| 26 | $i-C_5H_{11}$ | $C_2H_5$ | |
| 27 | $n-C_6H_{13}$ | H | 141-142 |
| 28 | $n-C_6H_{13}$ | $CH_3$ | 80- 81 |
| 29 | $n-C_6H_{13}$ | $C_2H_5$ | |
| 30 | $n-C_7H_{15}$ | H | 123-124 |
| 31 | $n-C_7H_{15}$ | $CH_3$ | 51- 52 |
| 32 | $n-C_7H_{15}$ | $C_2H_5$ | |
| 33 | $n-C_4H_9-CH(C_2H_5)-$ | H | |
| 34 | $n-C_4H_9-CH(C_2H_5)-$ | $CH_3$ | |
| 35 | $n-C_4H_9-CH(C_2H_5)-$ | $C_2H_5$ | |
| 36 | $n-C_8H_{17}$ | H | |
| 37 | $n-C_8H_{17}$ | $CH_3$ | 105-106 |
| 38 | $n-C_8H_{17}$ | $C_2H_5$ | |

Tabelle 1 (Fortsetzung)

| Verb.Nr. | $R^1$ | $R^2$ | Schmp. (°C) |
|---|---|---|---|
| 39 | $t-C_4H_9-CH_2-CH(CH_3)-CH_2-$ | H | |
| 40 | $t-C_4H_9-CH_2-CH(CH_3)-CH_2-$ | $CH_3$ | |
| 41 | $t-C_4H_9-CH_2-CH(CH_3)-CH_2-$ | $C_2H_5$ | |
| 42 | $n-C_9H_{19}$ | H | 122-123 |
| 43 | $n-C_9H_{19}$ | $CH_3$ | 72- 73 |
| 44 | $n-C_9H_{19}$ | $C_2H_5$ | |
| 45 | $n-C_{10}H_{21}$ | H | 133-134 |
| 46 | $n-C_{10}H_{21}$ | $CH_3$ | 59- 60 |
| 47 | $n-C_{10}H_{21}$ | $C_2H_5$ | |
| 48 | $n-C_{11}H_{23}$ | H | |
| 49 | $n-C_{11}H_{23}$ | $CH_3$ | |
| 50 | $n-C_{11}H_{23}$ | $C_2H_5$ | |
| 51 | $n-C_{12}H_{25}$ | H | |
| 52 | $n-C_{12}H_{25}$ | $CH_3$ | |
| 53 | $n-C_{12}H_{25}$ | $C_2H_5$ | |
| 54 | $n-C_4H_9-CH(C_2H_5)-CH_2$<br>$-(CH_2)_2-O$ | H | |
| 55 | $n-C_4H_9-CH(C_2H_5)-CH_2$<br>$-(CH_2)_2-O$ | $CH_3$ | |
| 56 | $n-C_4H_9-CH(C_2H_5)-CH_2$<br>$-(CH_2)_3-O$ | H | |
| 57 | $n-C_4H_9-CH(C_2H_5)-CH_2$<br>$-(CH_2)_3-O$ | $CH_3$ | |
| 58 | $n-C_4H_9-CH(C_2H_5)-CH_2$<br>$-(CH_2)_4-O$ | H | |
| 59 | $n-C_4H_9-CH(C_2H_5)-CH_2$<br>$-(CH_2)_5-O$ | H | |
| 60 | $t-C_4H_9-CH_2-CH(CH_3)-(CH_2)_2$<br>$-(CH_2)_2-O$ | H | |
| 61 | $t-C_4H_9-CH_2-CH(CH_3)-(CH_2)_2$<br>$-(CH_2)_3-O$ | H | |
| 62 | $t-C_4H_9-CH_2-CH(CH_3)-(CH_2)_2$<br>$-(CH_2)_4-O$ | H | |
| 63 | $C_6H_5-O-(CH_2)_2-$ | H | 189-190 |
| 64 | $C_6H_5-O-(CH_2)_2-$ | $CH_3$ | |
| 65 | $C_6H_5-O-(CH_2)_2-$ | $C_2H_5$ | |

Tabelle 1 (Fortsetzung)

| Verb.Nr. | $R^1$ | $R^2$ | Schmp. (°C) |
|---|---|---|---|
| 66 | $C_6H_5-O-(CH_2)_3-$ | H | 144-145 |
| 67 | $C_6H_5-O-(CH_2)_3-$ | $CH_3$ | |
| 68 | $C_6H_5-O-(CH_2)_4-$ | H | |
| 69 | $C_6H_5-O-(CH_2)_4-$ | $CH_3$ | |
| 70 | $C_6H_5-O-(CH_2)_5-$ | H | 142-143 |
| 71 | $C_6H_5-O-(CH_2)_5-$ | $CH_3$ | |
| 72 | $C_6H_5-O-(CH_2)_6-$ | H | |
| 73 | $C_6H_5-O-(CH_2)_6-$ | $CH_3$ | |
| 74 | $C_6H_5-O-(CH_2)_8-$ | H | |
| 75 | $C_6H_5-O-(CH_2)_8-$ | $CH_3$ | |
| 76 | $2-CH_3-C_6H_4-O-(CH_2)_2-$ | H | |
| 77 | $2-CH_3-C_6H_4-O-(CH_2)_2-$ | $CH_3$ | |
| 78 | $2-CH_3-C_6H_4-O-(CH_2)_3-$ | H | |
| 79 | $2-CH_3-C_6H_4-O-(CH_2)_3-$ | $CH_3$ | |
| 80 | $2-CH_3-C_6H_4-O-(CH_2)_4-$ | H | |
| 81 | $2-CH_3-C_6H_4-O-(CH_2)_5-$ | H | |
| 82 | $3-CH_3-C_6H_4-O-(CH_2)_2-$ | H | |
| 83 | $4-CH_3-C_6H_4-O-(CH_2)_2-$ | H | |
| 84 | $2,3-(CH_3)_2-C_6H_3-O-(CH_2)_2-$ | H | |
| 85 | $2,4-(CH_3)_2-C_6H_3-O-(CH_2)_2-$ | H | |
| 86 | $3,4-(CH_3)_2-C_6H_3-O-(CH_2)_2-$ | H | |
| 87 | $3,5-(CH_3)_2-C_6H_3-O-(CH_2)_2-$ | H | |
| 88 | $2,5-(CH_3)_2-C_6H_3-O-(CH_2)_2-$ | H | |
| 89 | $2,6-(CH_3)_2-C_6H_3-O-(CH_2)_2-$ | H | |
| 90 | $2-Cl-C_6H_4-O-(CH_2)_2-$ | H | 180-181 |
| 91 | $3-Cl-C_6H_4-O-(CH_2)_2-$ | H | |
| 92 | $4-Cl-C_6H_4-O-(CH_2)_2-$ | H | |
| 93 | $2,4-Cl_2-C_6H_3-O-(CH_2)_2-$ | H | |
| 94 | $3,5-Cl_2-C_6H_3-O-(CH_2)_2-$ | H | |
| 95 | $2-Cl-C_6H_4-O-(CH_2)_3-$ | H | |
| 96 | $3-Cl-C_6H_4-O-(CH_2)_3-$ | H | |
| 97 | $4-Cl-C_6H_4-O-(CH_2)_3-$ | H | |
| 98 | $2,4-Cl_2-C_6H_3-O-(CH_2)_3-$ | H | |
| 99 | $3,5-Cl_2-C_6H_3-O-(CH_2)_3-$ | H | |
| 100 | $2-Cl-C_6H_4-O-(CH_2)_4-$ | H | |
| 101 | $4-Cl-C_6H_4-O-(CH_2)_4-$ | H | |
| 102 | $2-Cl-C_6H_4-O-(CH_2)_5-$ | H | 123-124 |
| 103 | $4-Cl-C_6H_4-O-(CH_2)_5-$ | H | |
| 104 | $2-Cl-C_6H_4-O-(CH_2)_6-$ | H | |
| 105 | $4-Cl-C_6H_4-O-(CH_2)_6-$ | H | |
| 106 | $2-Cl-C_6H_4-O-(CH_2)_8-$ | H | |
| 107 | $4-Cl-C_6H_4-O-(CH_2)_8-$ | H | |

Tabelle 2

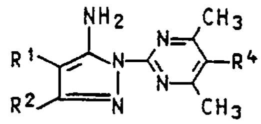

| Verb.Nr. | $R^1$ | $R^2$ | $R^4$ | Schmp. (°C) |
|---|---|---|---|---|
| 108 | $n-C_4H_9$ | H | H | |
| 109 | $i-C_4H_9$ | H | H | |
| 110 | $s-C_4H_9$ | H | H | |
| 111 | $t-C_4H_9$ | H | H | |
| 112 | $n-C_4H_9$ | $CH_3$ | H | |
| 113 | $i-C_4H_9$ | $CH_3$ | H | |
| 114 | $n-C_4H_9$ | $C_2H_5$ | H | |
| 115 | $i-C_4H_9$ | $C_2H_5$ | H | |
| 116 | $n-C_4H_9$ | H | $CH_3$ | |
| 117 | $n-C_4H_9$ | H | $C_2H_5$ | |
| 118 | $n-C_4H_9$ | H | $n-C_3H_7$ | |
| 119 | $n-C_4H_9$ | H | $n-C_4H_9$ | |
| 120 | $n-C_4H_9$ | H | $i-C_4H_9$ | |
| 121 | $n-C_5H_{11}$ | H | H | |
| 122 | $n-C_5H_{11}$ | $CH_3$ | H | |
| 123 | $n-C_5H_{11}$ | H | $CH_3$ | |
| 124 | $n-C_5H_{11}$ | H | $C_2H_5$ | |
| 125 | $n-C_5H_{11}$ | H | $n-C_3H_7$ | |
| 126 | $n-C_5H_{11}$ | H | $n-C_4H_9$ | |
| 127 | $n-C_5H_{11}$ | H | $i-C_4H_9$ | |
| 128 | $n-C_6H_{13}$ | H | H | 101-102 |
| 129 | $n-C_6H_{13}$ | $CH_3$ | H | |
| 130 | $n-C_6H_{13}$ | H | $CH_3$ | |
| 131 | $n-C_6H_{13}$ | H | $C_2H_5$ | |
| 132 | $n-C_6H_{13}$ | H. | $n-C_3H_7$ | |
| 133 | $n-C_7H_{15}$ | H | H | |
| 134 | $n-C_7H_{15}$ | $CH_3$ | H | |
| 135 | $n-C_7H_{15}$ | H | $CH_3$ | |
| 136 | $n-C_7H_{15}$ | $C_2H_5$ | H | |
| 137 | $n-C_7H_{15}$ | $n-C_3H_7$ | H | |
| 138 | $n-C_7H_{15}$ | H | $C_2H_5$ | |
| 139 | $n-C_7H_{15}$ | H | $n-C_3H_7$ | |
| 140 | $n-C_8H_{17}$ | H | H | |
| 141 | $n-C_8H_{17}$ | $CH_3$ | H | |
| 142 | $n-C_8H_{17}$ | H | $CH_3$ | |
| 143 | $n-C_8H_{17}$ | $C_2H_5$ | H | |
| 144 | $n-C_8H_{17}$ | $n-C_3H_7$ | H | |

Tabelle 2 (Fortsetzung)

| Verb.Nr. | $R^1$ | $R^2$ | $R^4$ | Schmp. (°C) |
|---|---|---|---|---|
| 145 | $n-C_8H_{17}$ | H | $C_2H_5$ | |
| 146 | $n-C_8H_{17}$ | H | $n-C_3H_7$ | |
| 147 | $n-C_4H_9-CH(C_2H_5)-$ | H | H | |
| 148 | $n-C_4H_9-CH(C_2H_5)-$ | $CH_3$ | H | |
| 149 | $n-C_4H_9-CH(C_2H_5)-$ | H | $CH_3$ | |
| 150 | $n-C_4H_9-CH(C_2H_5)-$ | H | $C_2H_5$ | |
| 151 | $n-C_9H_{19}$ | H | H | |
| 152 | $n-C_9H_{19}$ | $CH_3$ | H | |
| 153 | $n-C_9H_{19}$ | H | $CH_3$ | |
| 154 | $n-C_9H_{19}$ | H | $C_2H_5$ | |
| 155 | $n-C_9H_{19}$ | H | $n-C_3H_7$ | |
| 156 | $n-C_{10}H_{21}$ | H | H | 86- 87 |
| 157 | $n-C_{10}H_{21}$ | $CH_3$ | H | |
| 158 | $n-C_{10}H_{21}$ | H | $CH_3$ | |
| 159 | $n-C_{10}H_{21}$ | H | $C_2H_5$ | |
| 160 | $n-C_{11}H_{23}$ | H | H | |
| 161 | $n-C_{11}H_{23}$ | $CH_3$ | H | |
| 162 | $n-C_{11}H_{23}$ | H | $CH_3$ | |
| 163 | $n-C_{12}H_{25}$ | H | H | |
| 164 | $t-C_4H_9-CH_2-CH(CH_3)-CH_2-$ | H | H | |
| 165 | $t-C_4H_9-CH_2-CH(CH_3)-CH_2-$ | $CH_3$ | H | |
| 166 | $t-C_4H_9-CH_2-CH(CH_3)-CH_2-$ | H | $CH_3$ | |
| 167 | $t-C_4H_9-CH_2-CH(CH_3)-CH_2-$ | H | $C_2H_5$ | |
| 168 | $t-C_4H_9-CH_2-CH(CH_3)-CH_2-$ | H | $n-C_3H_7$ | |
| 169 | $t-C_4H_9-CH_2-CH(CH_3)-CH_2-$ | H | $i-C_3H_7$ | |
| 170 | $t-C_4H_9-CH_2-CH(CH_3)-CH_2-$ | H | $n-C_4H_9$ | |
| 171 | $t-C_4H_9-CH_2-CH(CH_3)-CH_2-$ | H | $i-C_4H_9$ | |
| 172 | $C_6H_5-O-(CH_2)_2-$ | H | H | |
| 173 | $C_6H_5-O-(CH_2)_2-$ | $CH_3$ | H | |
| 174 | $C_6H_5-O-(CH_2)_2-$ | H | $CH_3$ | |
| 175 | $C_6H_5-O-(CH_2)_2-$ | H | $C_2H_5$ | |
| 176 | $C_6H_5-O-(CH_2)_2-$ | H | $n-C_3H_7$ | |
| 177 | $C_6H_5-O-(CH_2)_2-$ | H | $n-C_3H_9$ | |
| 178 | $C_6H_5-O-(CH_2)_3-$ | H | H | |
| 179 | $C_6H_5-O-(CH_2)_4-$ | H | H | |
| 180 | $C_6H_5-O-(CH_2)_5-$ | H | H | |
| 181 | $C_6H_5-O-(CH_2)_6-$ | H | H | |
| 182 | $C_6H_5-O-(CH_2)_8-$ | H | H | |
| 183 | $2-CH_3-C_6H_4-O-(CH_2)_3-$ | H | H | |
| 184 | $2-CH_3-C_6H_4-O-(CH_2)_4-$ | H | H | |

Tabelle 2 (Fortsetzung)

| Verb.Nr. | $R^1$ | $R^2$ | $R^4$ | Schmp. (°C) |
|---|---|---|---|---|
| 185 | $2-CH_3-C_6H_4-O-(CH_2)_5-$ | H | H | |
| 186 | $4-CH_3-C_6H_4-O-(CH_2)_3-$ | H | H | |
| 187 | $4-CH_3-C_6H_4-O-(CH_2)_4-$ | H | H | |
| 188 | $4-CH_3-C_6H_4-O-(CH_2)_5-$ | H | H | |
| 189 | $2-Cl-C_6H_4-O-(CH_2)_3-$ | H | H | |
| 190 | $2-Cl-C_6H_4-O-(CH_2)_4-$ | H | H | |
| 191 | $2-Cl-C_6H_4-O-(CH_2)_5-$ | H | H | |
| 192 | $2-Cl-C_6H_4-O-(CH_2)_6-$ | H | H | |
| 193 | $2-Cl-C_6H_4-O-(CH_2)_8-$ | H | H | |
| 194 | $4-Cl-C_6H_4-O-(CH_2)_3-$ | H | H | |
| 195 | $4-Cl-C_6H_4-O-(CH_2)_4-$ | H | H | |
| 196 | $4-Cl-C_6H_4-O-(CH_2)_5-$ | H | H | |
| 197 | $4-Cl-C_6H_4-O-(CH_2)_6-$ | H | H | |
| 198 | $4-Cl-C_6H_4-O-(CH_2)_8-$ | H | H | |
| 199 | $n-C_4H_9-CH(C_2H_5)-CH_2$<br>$-(CH_2)_2-O$ | H | H | |
| 200 | $n-C_4H_9-CH(C_2H_5)-CH_2$<br>$-(CH_2)_3-O$ | H | H | |
| 201 | $n-C_4H_9-CH(C_2H_5)-CH_2$<br>$-(CH_2)_4-O$ | H | H | |
| 202 | $t-C_4H_9-CH_2-CH(CH_3)-(CH_2)_2$<br>$-(CH_2)_2-O$ | H | H | |
| 203 | $t-C_4H_9-CH_2-CH(CH_3)-(CH_2)_2$<br>$-(CH_2)_3-O$ | H | H | |
| 204 | $t-C_4H_9-CH_2-CH(CH_3)-(CH_2)_2$<br>$-(CH_2)_4-O$ | H | H | |
| 205 | $t-C_4H_9-CH_2-CH(CH_3)-(CH_2)_2$<br>$-(CH_2)_5-O$ | H | H | |

Die substituierten 5-Amino-pyrazole der Formel I zeichnen sich, allgemein ausgedrückt, durch eine hervorragende Wirksamkeit gegen ein breites Spektrum von pflanzenpathogenen Pilzen, insbesondere aus der Klasse der Ascomyceten, Basidiomyceten und Deuteromyceten, aber auch der Phycomyceten aus. Sie sind zum Teil systemisch wirksam und können als Blatt- und Bodenfungizide eingesetzt werden.

Von besonderem Interesse sind die Verbindungen der Formel I für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Reben sowie Gemüse, wie Gurken, Bohnen und Kürbisgewächse.

Die Verbindungen der Formel I sind insbesondere zur Bekämpfung folgender Pflanzenkrankheiten geeignet:

Phytophthora infestans an Tomaten und Kartoffeln,

Phytophthora parasitica an Erdbeeren,

Pseudoperonospora cubensis an Gurken,
Pseudoperonospora humuli an Hopfen,
Peronospora destructor an Zwiebeln,
Peronospora tabacina an Tabak,
Plasmopara viticola an Reben,
Plasmopara halstedii an Sonnenblumen,
Sclerospora macrospora an Mais,
Bremia lactucae an Salat,
Mucor mucedo an Früchten,
Rhizopus nigricans an Rüben sowie
Erysiphe graminis an Getreide,
Uncinula necator an Reben,
Sphaerotheca fuliginea an Kürbisgewächsen,
Puccinia-Arten an Getreide,
Rhizoctonia-Arten an Baumwolle,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis sowie
Fusarium- und Verticillium-Arten an verschiedenen Pflanzen.

Die Verbindungen der Formel I können auch im Materialschutz eingesetzt werden, z.B. gegen Paecilomyces variotii.

Die 5-Aminopyrazole werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Die Aufwandmengen betragen je nach Art des gewünschten Effektes zwischen 0,05 und 2 kg Wirkstoff oder mehr je ha.

Die Verbindungen der Formel I können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Beispiele für solche Zubereitungen sind:

I. Man vermischt 90 Gew.-Teile der Verbindung Nr. 30 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 27 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 43 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 27 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen

und feines Verteilen der Lösung in Wasser erhält man eine wäßrige Dispersion.

V. 80 Gew.-Teile der Verbindung Nr. 31 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 128 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 128 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 156 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 43 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergößerung des fungiziden Wirkungsspektrums.

Anwendungsbeispiele

Beispiel A

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Dieser Versuch ergibt, daß die Verbindungen Nr. 27, 30, 31, 43 und 128 bei Anwendung einer 0,05 %igen wäßrigen Wirkstoffaufbereitung eine gute fungizide Wirkung haben.

Beispiel B

Wirksamkeit gegen Plasmopara viticola

Blätter von Topfreben der Sorte "Müller-Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschmemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Dieser Versuch zeigt, daß die Verbindungen Nr. 27, 30, 31, 42, 43, 45, 128 und 156 bei Anwendung einer 0,05 %igen wäßrigen Spritzbrühe eine sehr gute fungizide Wirkung haben.

**Patentansprüche**

1. Substituierte 5-Amino-pyrazole der Formel I

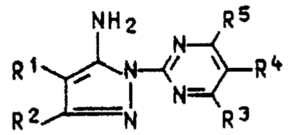

(I)

in der
$R^1$ einen geradkettigen oder verzweigten $C_4$-$C_{12}$-Alkyl- oder einen geradkettigen oder verzweigten $C_4$-$C_{12}$-Alkoxy-$C_1$-$C_5$-alkyl-rest oder einen gegebenenfalls durch $C_1$-$C_4$-Alkyl oder Halogen im Phenylrest oder Naphthylrest substituierten Phenoxy-$C_1$-$C_8$-alkylrest oder Naphthoxy-$C_1$-$C_8$-alkylrest,
$R^2$, $R^3$ und $R^5$ unabhängig voneinander Wasserstoff oder einen $C_1$-$C_4$-Alkylrest,

$R^4$ Wasserstoff, einen geradkettigen oder verzweigten $C_1$-$C_6$-Alkyl- oder einen geradkettigen oder verzweigten $C_1$-$C_6$-Alkoxy-$C_1$-$C_5$-alkylrest bedeuten.

2. Substituierte 5-Amino-pyrazole der Formel I gemäß Anspruch 1, dadurch gekennzeichnet, daß $R^3$, $R^4$ und $R^5$ Wasserstoff bedeuten.

3. Verfahren zur Herstellung von substituierten 5-Amino-pyrazolen der Formel I gemäß Anspruch 1, dadurch gekennzeichnet, daß man ein α-Acyl-nitril der Formel II,

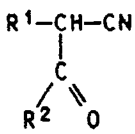

IIa

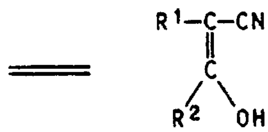

IIb

das entweder als Tautomer IIa oder IIb oder als Gemisch von beiden vorliegen kann, in an sich bekannter Weise mit einem substituierten Hydrazin der Formel III

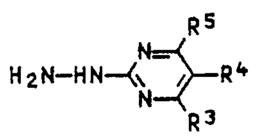

(III),

in einem geeigneten Lösungs- oder Verdünnungsmittel und gegebenenfalls in Gegenwart eines Katalysators zur Reaktion bringt.

4. Fungizides Mittel, enthaltend einen inerten Trägerstoff und ein substituiertes 5-Amino-pyrazol der Formel I

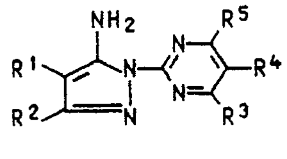

(I)

in der
$R^1$ einen geradkettigen oder verzweigten $C_4$-$C_{12}$-Alkyl- oder einen geradkettigen oder verzweigten $C_4$-$C_{12}$-Alkoxy-$C_1$-$C_5$-alkyl-rest oder einen gegebenenfalls durch $C_1$-$C_4$-Alkyl oder Halogen im Phenylrest oder Naphthylrest substituierten Phenoxy-$C_1$-$C_8$-alkylrest oder Naphthoxy-$C_1$-$C_8$-alkylrest,

$R^2$, $R^3$ und $R^5$ unabhängig voneinander Wasserstoff oder einen $C_1$-$C_4$-Alkylrest,

$R^4$ Wasserstoff, einen geradkettigen oder verzweigten $C_1$-$C_6$-Alkyl- oder einen geradkettigen oder verzweigten $C_1$-$C_6$-Alkoxy-$C_1$-$C_5$-alkylrest bedeuten.

5. Verfahren zur Bekämpfung von Pilzen, dadurch gekennzeichnet, daß man die Pilze oder die vor Pilzbefall zu schützenden Materialien, Pflanzen, Böden oder Saatgüter mit einer fungizid wirksamen Menge eines substituierten 5-Amino-pyrazols der Formel I

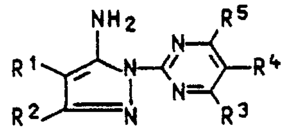

(I)

in der
$R^1$ einen geradkettigen oder verzweigten $C_4$-$C_{12}$-Alkyl- oder einen geradkettigen oder verzweigten $C_4$-$C_{12}$-Alkoxy-$C_1$-$C_5$-alkyl-rest oder einen gegebenenfalls durch $C_1$-$C_4$-Alkyl oder Halogen im Phenylrest oder Naphthylrest substituierten Phenoxy-$C_1$-$C_8$-alkylrest oder Naphthoxy-$C_1$-$C_8$-alkylrest,

$R^2$, $R^3$ und $R^5$ unabhängig voneinander Wasserstoff oder einen $C_1$-$C_4$-Alkylrest,

$R^4$ Wasserstoff, einen geradkettigen oder verzweigten C1-C6-Alkyl- oder einen geradkettigen oder verzweigten $C_1$-$C_6$-Alkoxy-$C_1$-$C_5$-alkylrest bedeuten, behandelt.

6. 5-Amino-4-n-hexyl-1-(pyrimidin-2-yl)-1H-pyrazol.

7. 5-Amino-4-n-heptyl-1-(pyrimidin-2-yl)-1H-pyrazol.

8. 5-Amino-4-n-heptyl-3-methyl-1-(pyrimidin-2-yl)-1H-pyrazol.

9. 5-Amino-4-n-hexyl-1-(4,6-dimethyl-pyrimidin-2-yl)-1H-pyrazol.

## Claims

1. A substituted 5-aminopyrazole of the formula I

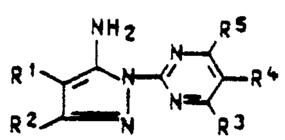

(I)

where
$R^1$ is straight-chain or branched $C_4$-$C_{12}$-alkyl or straight-chain or branched $C_4$-$C_{12}$-alkoxy-$C_1$-$C_5$-alkyl or is phenoxy-$C_1$-$C_8$-alkyl or naphthyloxy-$C_1$-$C_8$-alkyl which is unsubstituted or substituted in the phenyl radical or naphthyl radical by $C_1$-$C_4$-alkyl or halogen,
$R^2$, $R^3$ and $R^5$ independently of one another are each hydrogen or $C_1$-$C_4$-alkyl and
$R^4$ is hydrogen, straight-chain or branched $C_1$-$C_6$-alkyl or straight-chain or branched $C_1$-$C_6$-alkoxy-$C_1$-$C_5$-alkyl.

2. A substituted 5-aminopyrazole of the formula I as claimed in claim 1, wherein $R^3$, $R^4$ and $R^5$ are each hydrogen.

3. A process for the preparation of a substituted 5-aminopyrazole of the formula I as claimed in claim 1, wherein an α-acylnitrile of the formula II

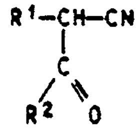

IIa

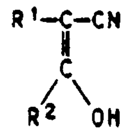

IIb

which may occur as either tautomer IIa or IIb or as a mixture of the two, is reacted in a conventional manner with a substituted hydrazine of the formula III

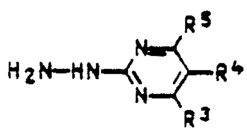

(III)

in a suitable solvent or diluent and in the presence or absence of a catalyst.

4. A fungicide containing an inert carrier and a substituted 5-aminopyrazole of the formula I

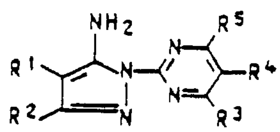

(I)

where
$R^1$ is straight-chain or branched $C_4$-$C_{12}$-alkyl or straight-chain or branched $C_4$-$C_{12}$-alkoxy-$C_1$-$C_5$-alkyl or is phenoxy-$C_1$-$C_8$-alkyl or naphthyloxy-$C_1$-$C_8$-alkyl which is unsubstituted or substituted in the phenyl radical or naphthyl radical by $C_1$-$C_4$-alkyl or halogen,
$R^2$, $R^3$ and $R^5$ independently of one another are each hydrogen or $C_1$-$C_4$-alkyl and
$R^4$ is hydrogen, straight-chain or branched $C_1$-$C_6$-alkyl or straight-chain or branched $C_1$-$C_6$-alkoxy-$C_1$-$C_5$-alkyl.

5. A method for controlling fungi, wherein the fungi or the materials, plants, soils or seeds to be protected from fungal attack are treated with a fungicidal amount of a substituted 5-aminopyrazole of the formula I

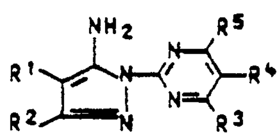

(I)

where
$R^1$ is straight-chain or branched $C_4$-$C_{12}$-alkyl or straight-chain or branched $C_4$-$C_{12}$-alkoxy-$C_1$-$C_5$-alkyl or is phenoxy-$C_1$-$C_8$-alkyl or naphthyloxy-$C_1$-$C_8$-alkyl which is unsubstituted or substituted in the phenyl radical or naphthyl radical by $C_1$-$C_4$-alkyl or halogen,
$R^2$, $R^3$ and $R^5$ independently of one another are each hydrogen or $C_1$-$C_4$-alkyl and
$R^4$ is hydrogen, straight-chain or branched $C_1$-$C_6$-alkyl or straight-chain or branched $C_1$-$C_6$-alkoxy-$C_1$-$C_5$-alkyl.

6. 5-Amino-4-n-hexyl-1-(pyrimidin-2-yl)-1H-pyrazole.

7. 5-Amino-4-n-heptyl-1-(pyrimidin-2-yl)-1H-pyrazole.

8. 5-Amino-4-n-heptyl-3-methyl-1-(pyrimidin-2-yl)-1H-pyrazole.

9. 5-Amino-4-n-hexyl-1-(4,6-dimethylpyrimidin-2-yl)-1H-pyrazole.

**Revendications**

1. 5-aminopyrazoles substitués de formule I

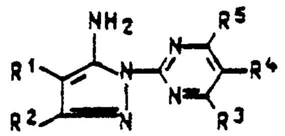

(I)

dans laquelle
$R^1$ représente un groupe alkyle en C 4-C 12 à chaîne droite ou ramifiée ou un groupe (alcoxy en C 4-C 12)-alkyle en C 1-C 5 à chaîne droite ou ramifiée ou un groupe phénoxy-alkyle en C 1-C 8 ou naphtoxy-alkyle en C 1-C 8 éventuellement substitué par des groupes alkyle en C 1-C 4 ou des halogènes dans le noyau phényle ou naphtyle,
$R^2$, $R^3$ et $R^5$ représentent chacun, indépendamment les uns des autres, l'hydrogène ou un groupe alkyle en C 1-C 4,
$R^4$ représente l'hydrogène, un groupe alkyle en C 1-C 6 à chaîne droite ou ramifiée ou un groupe (alcoxy en C 1-C 6)-alkyle en C 1-C 5 à chaîne droite ou ramifiée.

2. 5-aminopyrazoles substitués de formule I de la revendication 1, caractérisés en ce que $R^3$, $R^4$ et $R^5$ représentent l'hydrogène.

3. Procédé de préparation des 5-aminopyrazoles substitués de formule I de la revendication 1, caractérisé en ce que l'on fait réagir un alphaacylnitrile de formule II

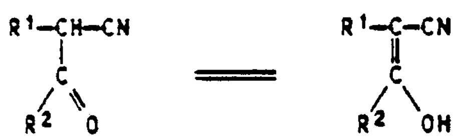

IIa IIb

à l'état du tautomère IIa ou du tautomère IIb ou à l'état de mélange des deux tautomères, de manière connue en soi, avec une hydrazine substituée de formule III

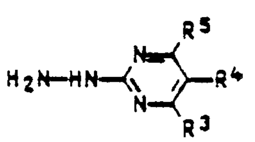

(III),

dans un solvant ou diluant approprié et éventuellement en présence d'un catalyseur.

4. Produit fongicide contenant un véhicule inerte et un 5-aminopyrazole substitué de formule I

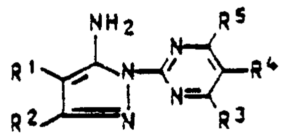

(I)

dans laquelle

$R^1$ représente un groupe alkyle en C 4-6 12 à chaîne droite ou ramifiée ou un groupe (alcoxy en C 4-C 12)-alkyle en C 1-6 5 à chaîne droite ou ramifiée, ou un groupe phénoxy-alkyle en C 1-C 8 ou naphtoxy-alkyle en C 1-C 8 éventuellement substitué par des groupes alkyle en C 1-C 4 ou des halogènes dans le noyau phényle ou dans le noyau naphtyle,

$R^2$, $R^3$ et $R^5$ représentent chacun, indépendamment les uns des autres, l'hydrogène ou un groupe alkyle en C 1-C 4,

$R^4$ représente l'hydrogène, un groupe alkyle en C 1-C 6 à chaîne droite ou ramifiée ou un groupe (alcoxy en C 1-6 6)-alkyle en C 1-6 5 à chaîne droite ou ramifiée.

**5.** Procédé pour combattre les mycètes, caractérisé en ce que l'on traite les mycètes ou les matériaux, végétaux, sols ou semences à protéger contre l'attaque par les mycètes par une quantité fongicide efficace d'un 5-aminopyrazole substitué de formule I

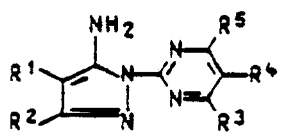

(I)

dans laquelle

$R^1$ représente un groupe alkyle en C 4-C 12 à chaîne droite ou ramifiée ou un groupe (alcoxy en C 4-C 12)-alkyle en C 1-C 5 à chaîne droite ou ramifiée ou un groupe phénoxy-alkyle en C 1-C 8 ou naphtoxy-alkyle en C 1-C 8 éventuellement substitué par des groupes alkyle en C 1-C 4 ou des halogènes dans le noyau phényle ou dans le noyau naphtyle,

$R^2$, $R^3$ et $R^5$ représentent chacun, indépendamment les uns des autres, l'hydrogène ou un groupe alkyle en C 1-C 4,

$R^4$ représente l'hydrogène, un groupe alkyle en C 1-C 6 à chaîne droite ou ramifiée ou un groupe (alcoxy en C 1-C 6)-alkyle en C 1-C 5 à chaîne droite ou ramifiée.

**6.** Le 5-amino-4-n-hexyl-1-(pyrimidine-2-yl)-1H-pyrazole.

**7.** Le 5-amino-4-n-heptyl-1-(pyrimidine-2-yl)-1H-pyrazole.

**8.** Le 5-amino-4-n-heptyl-3-méthyl-1-(pyrimidine-2-yl)-1H-pyrazole.

**9.** Le 5-amino-4-n-hexyl-1-(4,6-diméthylpyrimidine-2-yl)-1H-pyrazole.